# EUROPEAN PATENT APPLICATION

(11) **EP 2 990 429 A1**
(43) Date of publication of application: **02.03.2016**
(21) Application number: 15182566.8
(22) Date of filing: 26.08.2015
(51) Int. Cl.: C08G 18/79, C07D 229/00, C08G 18/02, C08G 18/72, C08G 18/76

(54) **PROCESS FOR THE PREPARATION OF LIGHT COLOURED, STABLE TO STORAGE URETONIMINE-MODIFIED POLYISOCYANATE**

(30) Priority: 29.08.2014 HU 1400402
(71) Applicant: Borsodchem Zrt, 3700 Kazincbarcika (HU)
(72) Inventor: LI, Wu, H-1037 Budapest (HU); TRUJILLO VILABOY, Jose, H-3525 Miskolc (HU); FEKETE, László, H-2100 Gödöll (HU)
(74) Representative: Schläfer, Laszlo

(57) **Abstract**

A process is disclosed for the preparation of light coloured, stable to storage uretonimine-modified polyisocyanates, wherein organic isocyanates based on one or more 4,4'-diphenyl-methane-diisocyanate (MDI) are partially carbodiimidized in the presence of a phospholene type catalyst, characterized in that
(a) the concentration of the catalyst based on the weight of the starting isocyanates is between 150 ppb and 1000 ppb;
(b) the reaction is performed at a temperature between 180 °C and 280 °C;
(c) at the intermediate, after achieving an isocyanate content between 24 to 27.5% the reaction is stopped by decreasing the reaction temperature below 100 °C.

## Description

The invention relates to a novel process for the preparation of uretonimine-modified polyphenyl-polymethylene-polyisocyanate with high storage stability and light colour. The essence of the process is the partial carbodiimidation of the isocyanate groups.

### The background of the invention:

According to US6120699 and EP515933 patent documents uretonimine-modified polyisocyanates may be readily prepared in the presence of phospholene, especially phospholene oxide based catalysts having high activity at moderate reaction temperature. Given the high activity of the catalyst, special care has to be taken during the stopping of the carbodiimidation reaction. Otherwise the uretonimine-modified polyisocyanates tend to slowly further react during their storage accompanied by the generation of carbon dioxide. Consequently, the pressure increases inside the closed storage device, and the isocyanate (NCO) content of the product permanently decreases.

A number of solutions have born for the stopping of the carbodiimidation reaction, one of the most often used among them is the addition of a so called catalyst poison or blocking agent to the reaction mixture. In DE2537685, EP0193787, US7030274 and US6120699 patent documents an acid, acid-chlorides, chloroformates and silicic acid are used as catalyst poison. The acid, such as perchloric acid has not been proved to be sufficiently effective.

A known and preferred catalyst poison is thionyl chloride, which, however, causes the yellowing of the stabilized uretonimine-modified polyisocyanates.

In EP515933 patent document trimethylsilyl trifluoro-methanesulphonate (TMST) was used as catalyst poison. However, it has been proven that the uretonimine-modified polyisocyanates prepared by using of TMST were not always stable.

In EP515933 patent document silicic acid is used in higher amount (50 to 100 times amount as compared to the weight of the catalyst) to block the phospholene catalyst. However, it was found that colour index of the uretonimine-modified polyisocyanates prepared in such a manner is considerably worse than that of the products prepared in another way.

WO2008/009669 patent document oxalyl chloride is proposed as catalyst poison, which is a toxic compound with 63°C boiling point. Due to the low boiling point of oxalyl chloride, special care should be taken in the course of manufacturing, as well as the use of the product.

In US2013012698 patent document a process is proposed for the preparation of stable to storage isocyanate mixture with low colour index, wherein the monoalkyl ester halogenides of oxalic acid are used as effective deactivating agents for the poisoning of the phospholene catalyst. Said compounds may be ethyl oxalyl chloride or methyl oxalyl chloride.

### Summary of the invention:

In the course of elaborating of the present invention our object was to elaborate of a process for the preparation of uretonimine-modified polyisocyanates, with the help of which the above-mentioned technological shortcomings may be overcome.

With the application of the present invention such uretonimine-modified polyisocyanates can be prepared, which are stable, and do not change their colour during storage, and do not need the application of dangerous or poisonous catalyst blocking agents.

During our experiments we have surprisedly found that there is a concentration range of the phospholene based catalysts, where, with applying a suitable temperature, they carbodiimidize the isocyanates with good efficacy, even in the presence of antioxidants, then cooling the mixture to the storage temperature, the process stops without the use of a catalyst poison.

Accordingly, the invention relates to a process for the preparation of uretonimine-modified polyphenyl-polymethylene-polyisocyanates light coloured and stable to storage, wherein one or more 4,4'-diphenyl-methane-diisocyanate (MDI) based organic isocyanates are partially carbodiimidized in the presence of a phospholene type catalyst, in such a manner that
(a) the concentration of the catalyst is between 150 ppb and 1000 ppb based on the weight of the starting isocyanates;
(b) the reaction is performed at a temperature between 180 °C and 280 °C;
(c) at the intermediate the reaction is stopped after reaching an isocyanate content between 24 to 27.5% by decreasing of the temperature to below 100 °C.

During the process a transparent uretonimine-modified polyisocyanate mixture can be prepared, having a yellow index of less than 10. The measurements were performed according to the ASTM D1925 standard using a UV-1008 Shimadzu spectrophotometer. The colour of the product is a lot more stable during storage, than in case of using catalyst poisons, even in case of higher storage temperature.

A further advantage of the invention is the abandoning of the catalyst poison, such as thionyl chloride, which causes the yellowing of the isocyanate, especially at high temperature.

A still further advantage is the complete abandoning of the use of dangerous, or even toxic products, such as oxalyl chloride.

### Detailed description of the invention:

In the process according to the invention the starting material is an organic isocyanate based on one or more 4,4'-diphenyl-methane-diisocyanate (MDI), wherein the one or more 4,4'-diphenyl-methane-diisocyanate is preferably 4,4'-diphenyl-methane-diisocyanate (MDI) or the mixture of its isomers, (4,4'-diphenyl-methane-diisocyanate ≥40%, 2,4-diphenyl-methane-diisocyanate ≤60%, 2,2-diphenyl-methane-diisocyanate ≤5%).

In the process according to the invention a variety of high activity phospholene type catalysts are used. It is preferred to use the phospholene oxide based, especially phospholene-1-oxide based catalysts, which have the following formula: 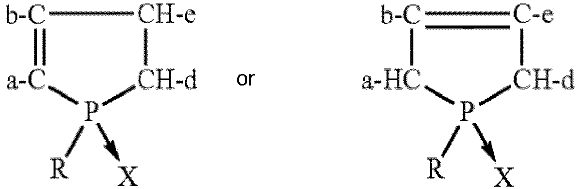 wherein a, b, d and e means each independently hydrogen or C1-C12 alkyl group, R means lower alkyl or aryl group, and X means oxygen or sulphur atom.

In the above context the C1-C12 alkyl group defined in the meaning of a, b, d and e means a saturated C1-C12 hydrocarbon group, such as, e.g. methyl, ethyl, propyl, butyl, pentyl, hexyl, heptyl, octyl, nonyl, decyl, undecyl or dodecyl group and the isomer derivatives thereof. In the meaning of R the lower alkyl group means a C1-C4 hydrocarbon group, such as, e.g. methyl, ethyl, n- or isopropyl, or n-, iso-, sec- or tert-butyl group. The aryl group preferably means phenyl group.

Characteristic compounds are as follows: 3-methyl-1-phenyl-3-phospholene-1-oxide, 3-methyl-1-phenyl-2-phospholene-1-oxide, 1-methyl-3-phospholene-1-oxide, 1-methyl-2-phospholene-1-oxide, 1-phenyl-3-phospholene-1-oxide and the mixtures of these isomers.

The catalyst may be used in a concentration between 150 ppb and 1000 ppb, preferably between 200 ppb and 800 ppb. The specific amount of the catalyst depends from the temperature of the reaction, the value of which is determined by the reaction duration to be achieved. It is preferred to use the catalyst at a concentration between 300 and 600 ppb based on the weight of the starting isocyanate. At this low concentration it was found that the activity of the catalyst at room temperature is extremely low, substantially insignificant. This makes it possible to stop the process after the formation of the carbodiimide groups during the manufacture by the cooling of the reaction mixture, in such a manner that the product be able to be stably stored at room temperature for prolonged period of time, without the chemical blocking of the catalyst.

Due to the extremely low concentration of the catalyst applied in the starting isocyanate, significantly high reaction temperature is necessary for the efficient carbodiimidation. At 115 °C or at a temperature below this value the activity of the catalyst is very low, insignificant. The reaction temperature is preferably set to a value between 190 °C and 240 °C, more preferably between 200 °C and 220 °C. The most preferred is to perform the reaction at 210 °C in order that the concentration of uretdione be kept at the lowest level in the end product. If the temperature of the reaction is kept within the range of 115 °C and 180 °C, the products will have a high uretdione concentration, which may very easily precipitate in the form of insoluble white particles during the storage and transport of the product. In case of too high reaction temperature the uretonimine-modified polyisocyanates will strongly coloured.

According to the present invention, the starting isocyanates may optionally comprise an additive, wherein the additive used is phosphoric acid ester or a sterically hindered amine based stabilizer, or a multifunctional antioxidant.

As examples of the included antioxidants the steric inhibited phenolic type antioxidants, such as, e.g. butyl hydroxy toluene (BHT), Irganox 1076, Irganox 1010, Irganox 1135 and Anox 135 may be mentioned. These compounds may be present in the starting polyisocyanate, and thus, during the preparation of the uretonimine-modified polyisocyanate according to the invention. These materials do not adversely affect the process. Otherwise, the antioxidants listed above may be added to the polyisocyanate immediately before the carbodiimidation or thereafter.

According to the present invention, optionally a second, unmodified polyisocyanate is also added to the prepared uretonimine-modified polyisocyanate reaction mixture. The addition of the unmodified polyisocyanate can be accomplished, once the reaction temperature had decreased under 100 °C. The composition of the a second, unmodified polyisocyanate may be similar to the first polyisocyanate, or may contain besides the pure 4,4'-MDI isomer (≥40%) also 2,4'-MDI isomer (≤60%) or 2,4'-MDI isomer (≤5%). The weight ratio of the added second polyisocyanate to the modified MDI may vary between 1:0.9 and 1:99, preferably between 20:80 and 50:50.

According to the present invention the isocyanate content of the uretonimine-modified polyisocyanate product is between 24-32 weight%, more preferably 26-31 weight%, based on the total weight of the product, while its viscosity is between 10 and 700 mPa-s, more preferably 20-250 mPa-s.

The thus prepared uretonimine-modified polyisocyanates can be used as raw material in the preparation of pre-polymers based on a polyether or polyester polyolester, furthermore, as isocyanate component in two-component polyurethane systems for the preparation of a variety of polyurethanes, such as elastomers, coatings, adhesives, further integrated, semi-solid and soft polyurethane foams.

The following examples illustrate the preparation of the uretonimine-modified polyisocyanates according to the present invention, without the intention to limit the scope thereof.

### Example 1 (comparative example)

1500 ppm Irganox 1076 and 5 ppm 3-methyl-1-phenyl-2-phospholene-1-oxide catalyst was added (in a 10% toluene solution) to 650 parts per weight of a mixture comprising about 98% 4,4'-MDI and 2 % 2,4'-MDI and other isomers (in the following pure 4,4 '-MDI). The reaction mixture was heated to 110 ° C and was maintained at this temperature for about 3 hours, until achieving the planned 27.5 % NCO value. Then the reaction mixture was cooled to 50°C by also adding 150 parts per weight pure 4,4'-MDI. Then 120 ppm thionyl chloride (SOCl₂) was added and the mixture was stirred for 0.5 hour at this temperature. Then 350 parts per weight of pure 4,4 '-MDI was added, and the reaction mixture was further stirred at 70 °C for 1 hour.

The yellow index of the end product was 11.4, its NCO content was 29.1%.

### Example 2 (comparative example)

The process described in Example 1 was repeated within identical reaction conditions except for adding as catalyst poison to the reaction mixture 100 ppm methyl oxalyl chloride (MOCl) in place of thionyl chloride. The end product was pale yellow, its yellow index was 14.6, and its NCO content was 28.5%.

### Example 3 (according to the invention)

650 parts per weight of pure 4,4'-MDI was mixed with 300 ppb 3-methyl-1-phenyl-2-phospholene-oxide catalyst (in a 10% toluene solution), and was heated to 200°C. The temperature was maintained about 4 hours until achieving the planned 27.5 % NCO value. The reaction mixture was cooled to 70 °C, then 450 parts per weight 4,4'-MDI was added thereto, and it was further stirred at 70 °C for 1 hour.

The yellow index of the end product was 9.3, its NCO content was 28.57%.

### Example 4 (according to the invention)

The process described in Example 3 was repeated within identical reaction conditions except that the concentration of the catalyst was modified as follows:
4/1 The concentration of the catalyst was 0.5 ppm, and the reaction duration was about 3 hours. The yellow index of the end product was 9.31, while its NCO content was 28.8%.
4/2 The concentration of the catalyst was 0.75 ppm and the reaction duration was about 1.3 hours. The yellow index of the end product was 9.2, while its NCO content was 28.44%.
4/3 The concentration of the catalyst was 1 ppm, and the reaction duration was about 0.75 hours. The yellow index of the end product was 9.3, while its NCO content was 28.21%.

### Example 5 (comparative example)

650 parts per weight of pure 4,4'-MDI was mixed with 6.5 parts per weight tributyl phosphate (TBP) catalyst, and was heated to 200°C. This temperature was maintained for about 8 hours until achieving the planned 30.5 % NCO value. The reaction mixture was cooled 45 °C, and it was further stirred at this temperature for 24 hours.

The yellow index of the end product was 17.2, while its NCO content was 28.2%.

The end products were stored at 90 °C for 3 days in order to determine the storage stability. The yellow index and NCO content of the samples were measured before and after the heat treatment. The comparative results are summarized in Table 1.

**Table 1:**

| No. of Example | Temperature (°C) | Catalyst | | Catalyst poison | | Storage stability test (90°C/3 days) | | | |
|---|---|---|---|---|---|---|---|---|---|
| | | compound | conc. (ppm) | compound | conc. (ppm) | NCO change | Yellow Index | | |
| | | | | | | | before | after | change (%) |
| 1 | 110 | PhO | 5 | SOCl₂ | 120 | 0.8 | 11.4 | 44.1 | 287 |
| 2 | 110 | PhO | 5 | MOCl | 100 | 0.8 | 14.6 | 26.9 | 84 |
| 3 | 205 | PhO | 0.3 | - | - | 0.55 | 9.2 | 10.1 | 10 |
| 4/1 | 205 | PhO | 0.5 | - | - | 0.69 | 9.3 | 10.1 | 9 |
| 4/2 | 205 | PhO | 0.75 | - | - | 0.91 | 9.2 | 10.3 | 12 |
| 4/3 | 205 | PhO | 1 | - | - | 1.6 | 9.2 | 10.2 | 11 |
| 5 | 205 | TBP | 1(%) | - | - | 0.52 | 17.2 | 19.3 | 12 |

It is apparent from the data according to Table 1 that the end product yellow indices according to the products prepared in Examples 3 and 4, are significantly lower both before, and after the heat treatment, than those of the end products prepared using as catalyst poison thionyl chloride (Example 1) or methyl oxalyl chloride (Example 2). The best storage stability was achieved with the product prepared in Example 5 using TBP. However, due to the reaction performed at high temperature and long period of time, the yellow index of the product is the highest.

## Claims

1. A process for the preparation of light coloured, stable to storage uretonimine-modified polyisocyanates, wherein organic isocyanates based on one or more 4,4'-diphenyl-methane-diisocyanate (MDI) are partially carbodiimidized in the presence of a phospholene type catalyst, **characterized in that**
(a) the concentration of the catalyst based on the weight of the starting isocyanates is between 150 ppb and 1000 ppb;
(b) the reaction is performed at a temperature between 180 °C and 280 °C;
(c) at the intermediate, after achieving an isocyanate content between 24 to 27.5% the reaction is stopped by decreasing the reaction temperature below 100 °C.

2. The process as claimed in Claim 1, **characterized in that** the catalyst is phospholene oxide, which has the formula below: 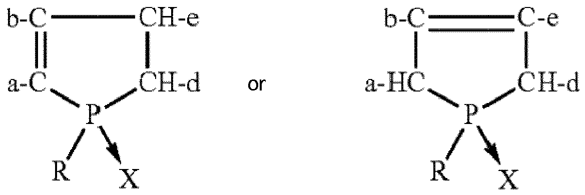 wherein a, b, d and e means each independently hydrogen or C1-C12 alkyl group, R means lower alkyl or aryl group, and X means oxygen or sulphur.

3. The process as claimed in Claim 2, **characterized in that** the catalyst is 3-methyl-1-phenyl-3-phospholene-1-oxide, 3-methyl-1-phenyl-2-phospholene-1-oxide, 1-methyl-3-phospholene-1-oxide, 1-methyl-2-phospholene-1-oxide, 1-phenyl-3-phospholene-1-oxide and the mixture of these isomers.

4. The process as claimed in any of Claims 1 to 3, **characterized in that** the concentration of the catalyst is preferably 200-800 ppb.

5. The process as claimed in any of Claims 1 to 4, **characterized in that** the reaction is performed at a temperature between 190-240°C, preferably between 200-220°C.

6. The process as claimed in any of Claims 1 to 5, **characterized in that** the starting isocyanates include an additive, wherein the used additive is phosphoric acid ester or a steric inhibited amine based stabilizer or a multifunctional antioxidant.

7. The process as claimed in any of Claims 1 to 6, **characterized in that** after the stopping of the reaction the reaction mixture is mixed with an unmodified polyisocyanate.

8. Light coloured, stable to storage uretonimine-modified polyisocyanate, which is obtainable by the process as claimed in any of Claims 1 to 7.

9. The light coloured, stable to storage uretonimine-modified polyisocyanate as claimed in Claim 8 for use in the preparation of pre-polymers based on a polyether or polyester polyol, further a variety of polyurethanes, such as elastomers, coatings, adhesives or integrated, semi solid and soft polyurethane foams.

10. Use of the light coloured, stable to storage uretonimine-modified polyisocyanate as claimed in Claim 8 in the preparation of pre-polymers based on a polyether or polyester polyol, further a variety of polyurethanes, such as elastomers, coatings, adhesives or integrated, semi solid and soft polyurethane foams.
